# EUROPÄISCHE PATENTSCHRIFT

(11) **EP 2 934 359 B1**
(45) Veröffentlichungstag und Bekanntmachung des Hinweises auf die Patenterteilung: **19.09.2018**
(21) Anmeldenummer: 13817892.6
(22) Anmeldetag: 03.12.2013
(51) Int. Cl.: A61B 90/50, A61B 90/57, A61B 17/00

(54) **INSTRUMENTENHALTER**
INSTRUMENT HOLDER
PORTE-INTRUMENT

(30) Priorität: 20.12.2012 DE 102012112712
(43) Veröffentlichungstag der Anmeldung: 28.10.2015
(73) Patentinhaber: Maquet GmbH, 76437 Rastatt (DE)
(72) Erfinder: WYSLUCHA, Ulrich, 76356 Weingarten (DE); PETER, Stefan, 76437 Rastatt (DE)
(74) Vertreter: Zacco GmbH
(86) Internationale Anmeldenummer: PCT/EP2013/075332
(87) Internationale Veröffentlichungsnummer: WO 2014/095336

(56) Entgegenhaltungen:
- DE-A1-102009 009 575
- US-A1- 2002 026 190
- US-A1- 2004 143 153
- US-A1- 2010 298 648
- US-B1- 6 283 912

## Beschreibung

Die vorliegende Erfindung betrifft einen Instrumentenhalter zum Befestigen eines medizinischen Instrumentes an einem Gelenkarm, mit einem Körper, einer Halterung zum Halten des medizinischen Instrumentes an dem Körper und einem Ankoppelelement zum Anbringen des Körpers an dem Gelenkarm.
Für chirurgische Anwendungen kommen heutzutage vermehrt Assistenzsysteme zum Einsatz, die einen Gelenkarm beinhalten, der beispielsweise an der Gleitschiene eines OP-Tisches anbringbar ist. An das freie Ende des frei beweglichen Gelenkarms wird ein medizinisches Instrument angekoppelt. Beispielsweise wird in der Laparoskopie ein mit einer Optik ausgestattetes starres Endoskop an dem Gelenkarm montiert und so ausgerichtet, dass das zu untersuchende oder chirurgisch zu versorgendes Zielgebiet von dem Operateur eingesehen werden kann.

US2004/0143153 offenbart einen Instrumentenhalter nach dem Oberbegriff des Anspruchs 1. Zusätzlich zu einem solchen Assistenzsystem kommen in der Chirurgie in der Regel weitere Instrumente zur Anwendung, die der Operateur oder ein Assistent von Hand bedienen muss. Ein Beispiel hierfür ist etwa ein Wundhaken, den der Assistent in die Wunde hält und mit einer Hand festhält, um das Operationsfeld für den Operateur zugänglich zu halten. Das Halten des Wundhakens ist für den Assistenten durchaus anstrengend, insbesondere wenn der Wundhaken über längere Zeit in ein und derselben Stellung gehalten werden muss.

Aufgabe der Erfindung ist es, eine Möglichkeit zu schaffen, ein medizinisches Instrument über längere Zeit einfach und stabil in der gewünschten Stellung zu halten, ohne dass hierfür medizinisches Personal benötigt wird.

Die Erfindung löst diese Aufgabe durch den Instrumentenhalter mit den Merkmalen des Anspruchs 1. Vorteilhafte Weiterbildungen sind in den Unteransprüchen vorgesehen.

Die Erfindung sieht einen Instrumentenhalter vor, der einen Körper, ein Ankoppelelement zur Anbringung des Körpers an einem Gelenkarm und eine Halterung zum Halten des medizinischen Instrumentes an dem Körper aufweist. Erfindungsgemäß umfasst die Halterung einen mit einem Außengewinde versehenen Gewindeschaft, eine Aufnahmescheibe und ein mit einem Innengewinde versehenes Verriegelungselement. Der Gewindeschaft steht von einer an dem Körper des Instrumentenhalters ausgebildeten ersten Anlagefläche ab. Die Aufnahmescheibe hat ein Durchgangsloch, über das die Aufnahmescheibe auf den Gewindeschaft steckbar und um den Gewindeschaft drehbar ist. Die Aufnahmescheibe hat ferner eine zweite Anlagefläche, die bei auf dem Gewindeschaft gesteckter Aufnahmescheibe der ersten Anlagefläche zugewandt ist, und eine Aufnahmenut, die zur Aufnahme eines Teils des medizinischen Instrumentes auf einer der zweiten Anlagefläche abgewandten Seite der Aufnahmescheibe ausgebildet ist. Das Verriegelungselement bedeckt im Gewindeeingriff seines Innengewindes mit dem Außengewinde des Gewindeschafts die Aufnahmenut zumindest teilweise, wodurch der genannte Teil des medizinischen Instrumentes in der Aufnahmenut gehalten ist, und ist zur kraftschlüssigen Anlage der beiden Anlageflächen gegen die Aufnahmescheibe festziehbar.

Der erfindungsgemäße Instrumentenhalter bildet somit eine mechanische Schnittstelle zwischen dem an dem OP-Tisch angebrachten Gelenkarm und dem medizinischen Instrument. Zur Befestigung des medizinischen Instrumentes wird zunächst der Instrumentenhalter über sein Ankoppelelement an dem Gelenkarm angebracht und dann das Instrument an dem Instrumentenhalter befestigt. Hierzu wird die Aufnahmescheibe über ihr Durchgangsloch auf den Gewindeschaft gesteckt und dann das Verriegelungselement auf den Gewindeschaft geschraubt. Da das Verriegelungselement, sobald es sich im Gewindeeingriff mit dem Gewindeschaft befindet, die Aufnahmenut zumindest teilweise bedeckt, ist der Teil des Instrumentes, der in der Aufnahmenut angeordnet ist, in der Aufnahmenut fixiert. Wird das Verriegelungselement weiter auf den Gewindeschaft geschraubt, so kommt die an dem Körper des Instrumentenhalters ausgebildete erste Anlagefläche in Kontakt mit der an der Aufnahmescheibe ausgebildeten zweiten Anlagefläche. Indem die Aufnahmescheibe mit dem Verriegelungselement auf dem Gewindeschaft festgezogen wird, werden die beiden Anlageflächen kraftschlüssig aufeinander gepresst, wodurch das medizinische Instrument in der gewünschten Stellung fixiert wird. Soll das Instrument verstellt werden, so muss das Verriegelungselement lediglich etwas gelöst werden, so dass die kraftschlüssige Anlage der beiden Anlageflächen aufgehoben wird. In diesem gelösten Zustand, in dem sich die Aufnahmescheibe immer noch im Gewindeeingriff mit dem Gewindeschaft befindet und somit den in der Aufnahmenut angeordneten Instrumententeil bedeckt, kann das Instrument frei um den Gewindeschaft gedreht werden. Sobald die gewünschte Stellung des Instrumentes eingestellt ist, wird das Verriegelungselement im Gewindeeingriff mit dem Gewindeschaft wieder gegen die Aufnahmescheibe festgezogen.

Der erfindungsgemäße Instrumentenhalter ermöglicht es also, das Instrument in besonders einfacher und zuverlässiger Weise nach Wunsch zu positionieren und dann in der gewünschten Position zu fixieren. Insbesondere wenn das Instrument über längere Zeit in ein- und derselben Position gehalten werden soll, erleichtert so der Instrumentenhalter die Handhabung des Instrumentes erheblich.

Das medizinische Instrument, das mittels des erfindungsgemäßen Instrumentenhalters befestigt wird, ist vorzugsweise ein Wundhaken, z.B. ein sogenannter Langenbeck-Wundhaken, der einstückig aus einem geraden Hakenschaft und einem rechtwinkelig abgebogenen Hakenblatt gebildet ist.
Der Instrumentenhalter wird vorzugsweise in Spritzgusstechnik gefertigt. So können beispielsweise der Körper des Instrumentenhalters mitsamt dem an dem Körper ausgebildeten Gewindeschaft, die Aufnahmescheibe und das Verriegelungselement jeweils als einstückiges Kunststoff-Spritzgussteil hergestellt werden, die dann zur Befestigung des Instrumentes an dem Gelenkarm zusammengesetzt werden.
In einer bevorzugten Ausführungsform ist die Aufnahmenut aus zwei geraden, jeweils an beiden Nutenden offenen Teilnuten gebildet, wobei das Durchgangsloch der Aufnahmescheibe zwischen den beiden Teilnuten angeordnet ist. Die beiden Teilnuten ermöglichen eine besonders zuverlässige Fixierung des medizinischen Instrumentes in der Aufnahmescheibe.
Vorzugsweise sind die beiden Teilnuten so angeordnet, dass sich ihre Verlängerungen außerhalb der Aufnahmescheibe in einem spitzen Winkel, der beispielsweise in einem Bereich von 10° bis 30° liegt, schneiden.

Erfindungsgemäß ist an der Aufnahmescheibe eine Nase ausgebildet, die vom Rand der Aufnahmescheibe seitlich bzw., wenn die Aufnahmescheibe kreisrund ausgeführt ist, radial übersteht und ausgebildet ist, einen U-förmigen Abschnitt des medizinischen Instrumentes zwischen sich und dem Körper des Instrumentenhalters zu halten. Ist die Aufnahmenut, wie weiter oben beschrieben, aus zwei geraden, jeweils an beiden Nutenden offenen Teilnuten gebildet, so befindet sich die Nase vorzugsweise in einem Bereich, der zwischen den Verlängerungen der beiden Teilnuten liegt. Sind die Teilnuten nicht-parallel angeordnet, so befindet sich die Nase vorzugsweise in einem Bereich, in dem die Verlängerungen der beiden Teilnuten auseinanderlaufen. Diese Ausgestaltung der Aufnahmescheibe ist auf ein medizinisches Instrument ausgelegt, dessen in der Aufnahmescheibe anzuordnender Instrumententeil durch einen U-förmigen Abschnitt gegeben ist, dessen beide Schenkel ausgehend von der Basis dieses Abschnittes zusammenlaufen. In diesem Fall können die beiden Schenkel des U-förmigen Abschnittes einfach derart in die beiden Teilnuten eingelegt werden, dass die Basis, welche die beiden Schenkel verbindet, in der Draufsicht auf die Aufnahmescheibe außerhalb der an der Aufnahmescheibe ausgebildeten Nase angeordnet ist. Anschließend wird das Instrument in die Richtung gezogen, in der die beiden Schenkel des U-förmigen Abschnittes zusammenlaufen. Dadurch gelangt die Basis des U-förmigen Abschnittes in einem Bereich unterhalb der Nase und wird in einem Raum, der zwischen der Nase und dem Körper des Instrumentenhalters, auf dem die Aufnahmescheibe angeordnet ist, fixiert. Selbst wenn das Verriegelungselement noch nicht auf den Gewindeschaft geschraubt ist, kann so sichergestellt werden, dass sich der U-förmige Abschnitt nicht von der Aufnahmescheibe löst.

Vorzugsweise hat das Verriegelungselement einen Sterngriff, mit dem das Verriegelungselement von Hand auf den Gewindeschaft schraubbar ist. Auf diese Weise lässt sich besonders einfach die kraftschlüssige Pressverbindung zwischen den beiden Anlageflächen herstellen und wieder lösen, um das medizinische Instrument an dem Instrumentenhalter zu fixieren bzw. in die gewünschte Stellung zu schwenken.

Vorzugsweise hat die erste Anlagefläche eine erste Zahnung und die zweite Anlagefläche eine zweite Zahnung, die bei der Anlage der beiden Anlageflächen ineinander greifen. Durch die beiden ineinander greifenden Zahnungen können die Anlageflächen besonders zuverlässig in verdrehsicheren Kontakt miteinander gebracht werden.

Vorteilhaft weisen die beiden Zahnungen jeweils mehrere längs eines Kreises angeordnete Zähne auf, die jeweils ein dreieckiges Zahnprofil haben. Die kreisförmige Anordnung der Zähne sowie deren dreieckiges Zahnprofil ermöglichen es, die Aufnahmescheibe in besonders einfacher Weise auf dem Körper des Instrumentenhalters zu drehen und damit den an der Aufnahmescheibe gehaltenen Instrumententeil zu verschwenken, wenn das Verriegelungselement zwar im Gewindeeingriff mit dem Gewindeschaft steht, jedoch nicht fest an dem Gewindeschaft festgezogen ist. In diesem Zustand gleiten die beiden Zahnungen infolge ihres dreieckigen Zahnprofils reibungsarm übereinander, wenn das Instrument verschwenkt und damit die Aufnahmescheibe auf dem Körper des Instrumentenhalters gedreht wird.

Dieses Zahnprofil bildet jedoch nur eine beispielhafte Ausgestaltung. Geeignet ist z.B. auch ein wellenförmiges Profil.

Vorzugsweise ist in dem Durchgangsloch der Aufnahmescheibe mindestens ein erstes Rastelement angeordnet, das beim Aufstecken der Aufnahmescheibe auf den Gewindeschaft mit mindestens einem an dem Gewindeschaft ausgebildeten zweiten Rastelement in Eingriff kommt. Beispielsweise ist das erste Rastelement ein Raststift mit einer in dem Durchgangsloch der Aufnahmescheibe radial nach innen weisenden Rastnase, während das zweite Rastelement eine ringförmig an dem Gewindeschaft ausgebildete Rastrille ist. Durch die beiden Rastelemente ist die Aufnahmescheibe im aufgesteckten Zustand auf dem Gewindeschaft gehalten und zugleich um diesen drehbar. Die Rastelemente bilden demnach eine Verliersicherung.

Die Erfindung sieht ferner eine medizinische Assistenzeinrichtung vor, die einen Gelenkarm, mindestens ein medizinisches Instrument und einen Instrumentenhalter vorstehend beschriebener Art umfasst.

Vorzugsweise umfasst die Assistenzeinrichtung eine Vielzahl von Instrumenten, die jeweils einen baugleichen, in der Aufnahmenut des Instrumentenhalters aufnehmbaren Instrumententeil aufweisen. Somit lässt sich mit ein- und demselben Instrumentenhalter eine Vielzahl von Instrumenten wahlweise mit dem Gelenkarm koppeln.

Die Erfindung wird im Folgenden anhand der Figuren näher erläutert. Darin zeigen:
- Fig. 1: eine erfindungsgemäße Assistenzeinrichtung mit einem Gelenkarm, einem Instrumentenhalters und einem Wundhaken, der über den Instrumentenhalter an dem Gelenkarm befestigt ist;
- Fig. 2: eine Darstellung, die den an dem Instrumentenhalter angebrachten Wundhakten ohne Gelenkarm zeigt;
- Fig. 3: eine auseinandergezogene Darstellung, welche die einzelnen Komponenten des Instrumentenhalters zeigt;
- Fig. 4: eine der Figur 3 entsprechende Darstellung aus einer anderen Perspektive; und
- Fig. 5: eine Darstellung einer in dem Instrumentenhalter vorgesehenen Aufnahmescheibe.

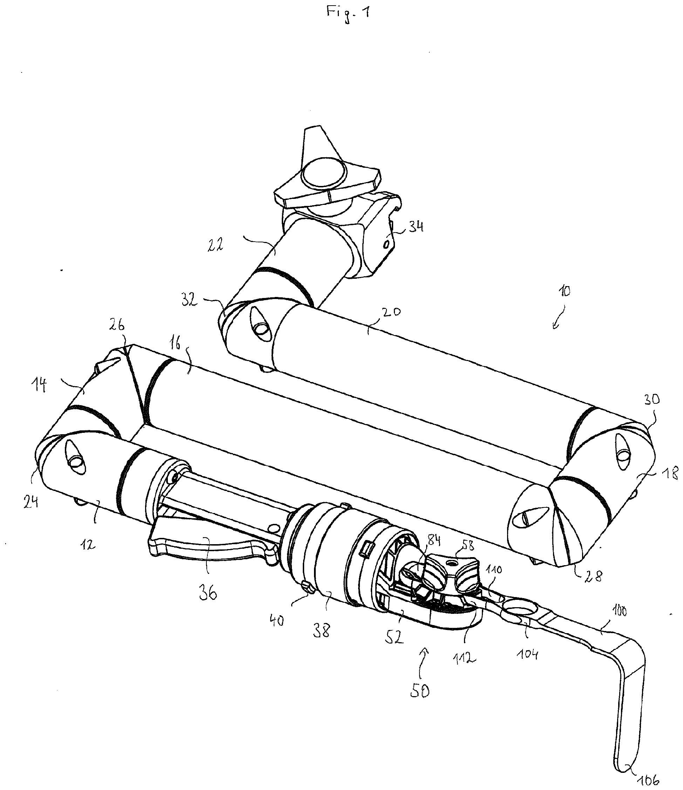

Figur 1 zeigt eine Assistenzeinrichtung mit einem Gelenkarm 10, der mehrere starre Halteglieder 12, 14, 16, 18, 20 und 22 aufweist, die über Gelenke 24, 26, 28, 30 und 32 miteinander gekoppelt sind. An einem Ende des Gelenkarms 10 ist eine Befestigungsvorrichtung 34 angeordnet, die dazu dient, den Gelenkarm 10 an einer nicht gezeigten Gleitschiene eines OP-Tisches anzubringen. An dem anderen Ende des Gelenkarms 10 befindet sich ein Haltegriff 36, der von dem Benutzer manuell betätigt werden kann, um den Gelenkarm 10 zu entriegeln.

Wird keine Betätigungskraft auf den Handgriff 36 ausgeübt, so sind die Halteglieder 12, 14, 16, 18, 20 und 22 des Gelenkarms 10 über die Gelenke 24, 26, 28, 30 und 32 starr miteinander gekoppelt. In diesem Zustand bildet der Gelenkarm eine starre Einheit.

Drückt der Benutzer den Handgriff 36, so werden über die Gelenke 24, 26, 28, 30 und 32 miteinander gekoppelten Halteglieder 12, 14, 16, 18, 20 und 22 über eine Entriegelungsmechanik, die nicht Teil der vorliegenden Erfindung ist und deshalb an dieser Stelle nicht näher beschrieben wird, gegeneinander beweglich, so dass der Benutzer den Gelenkarm 10 nach Wunsch im Raum ausrichten kann. Lässt der Benutzer anschließend den Handgriff 36 wieder los, so werden die Gelenke 24, 26, 28, 30 und 32 verriegelt, und der Gelenkarm 10 wird in seiner veränderten Ausrichtung fixiert.

An dem Ende des Gelenkarms 10, an dem sich der Haltegriff 36 befindet, ist eine Schnellkupplung 38 mit dem Gelenkarm 10 gekoppelt. Die Schnellkupplung 38 weist mehrere Entriegelungsknöpfe 40 auf, die gedrückt werden, um die Schnellkupplung 38 von dem Gelenkarm 10 zu lösen.

Über die Schnellkupplung 38 ist ein medizinisches Instrument, in dem vorliegenden Ausführungsbeispiel ein Langenbeck-Wundhaken 100, mit dem Gelenkarm 10 koppelbar. Hierzu ist ein in Figur 1 allgemein mit 50 bezeichneter Instrumentenhalter als Zwischenstück vorgesehen, der mit der Schnellkupplung 38 gekoppelt wird und den Wundhaken 100 hält. Der Gelenkarm 10 mitsamt der Schnellkupplung 38, der Instrumentenhalter 50 und der Wundhaken 100 bilden somit eine Einrichtung, die an dem OP-Tisch anbringbar ist und verwendet werden kann, das Wundfeld für den Operateur zugänglich zu halten, indem der Wundhaken 100 in die Wunde eingebracht und dort in der gewünschten Stellung fixiert wird.

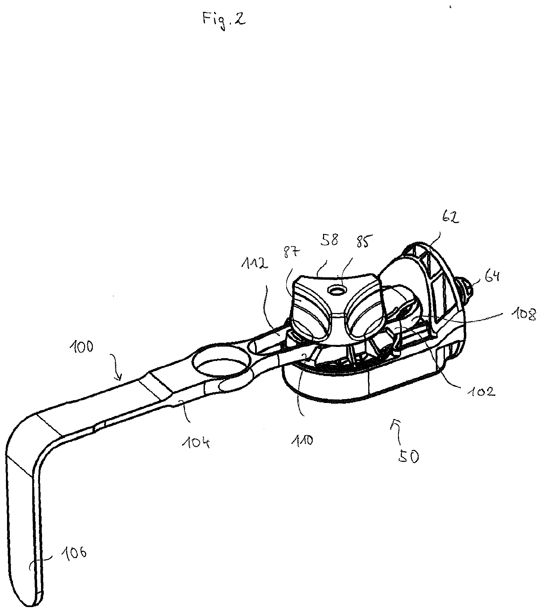

Figur 2 zeigt den Instrumentenhalter 50 mit dem an ihm angebrachten Wundhaken 100 in Alleinstellung, d.h. ohne den Gelenkarm 10 und die Schnellkupplung 38. Wie in Figur 2 gezeigt, ist der Wundhaken 100 ein einstückiges Bauteil, das einen U-förmigen Endabschnitt 102, einen an den Endabschnitt 102 anschließenden Hakenschaft 104 sowie ein rechtwinklig abgebogenes Hakenblatt 106 aufweist. Der U-förmige Abschnitt 102 ist aus einer Basis 108 sowie zwei an die Basis 108 anschließende Schenkel 110 und 112 gebildet. Die Schenkel 110 und 112 liegen in einer Ebene, sind jedoch nicht-parallel zueinander angeordnet, indem sie in der der genannten Ebene ausgehend von der Basis 108 zusammenlaufen.

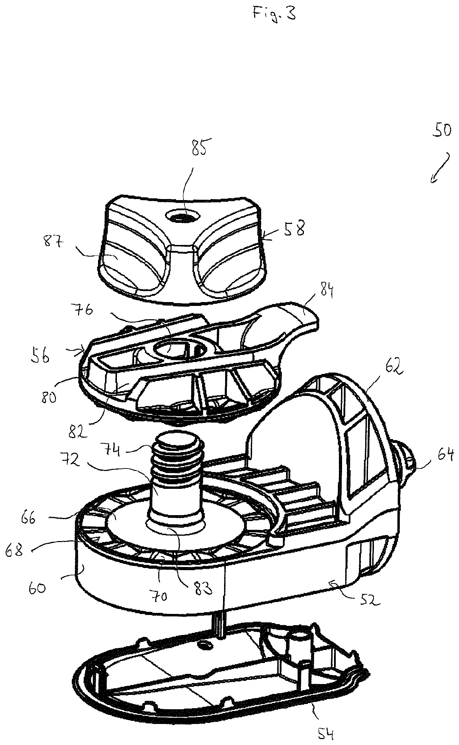

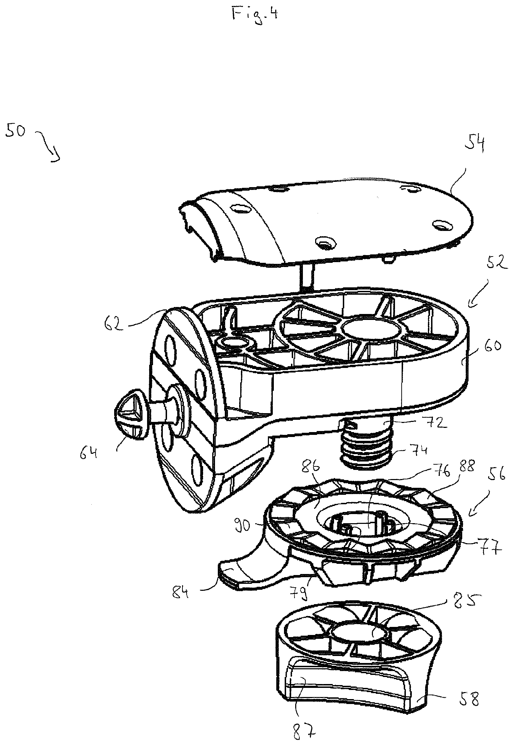

In den Figuren 3 und 4 sind die einzelnen Komponenten des Instrumentenhalters 100 in auseinander gezogenen Darstellungen und unterschiedlichen Perspektiven dargestellt.

Der Instrumentenhalter 50 besteht aus einem Körper 52, einer Abdeckung 54, die an der Unterseite des Körpers 52 aufgesteckt wird, einer Aufnahmescheibe 56 und einem Verriegelungselement 58. Der Körper 52, die Abdeckung 54, die Aufnahmescheibe 56 und das Verriegelungselement 58 sind jeweils als einstückiges Kunststoff-Spritzgussteil gefertigt.

Der Körper 52 weist eine Bodenplatte 60 und eine kreisrunde Rastplatte 62 auf, von der ein Rastzapfen 64 absteht. Der Rastzapfen 64 bildet ein Ankoppelelement, über das der Körper 52 an die Schnellkupplung 38 angekoppelt wird. An der Oberseite der Bodenplatte 60 ist eine erste Anlagefläche 66 ausgebildet, die eine erste umlaufende Zahnung 68 aufweist. Die Zahnung 68 besteht aus mehreren (in dem vorliegenden Ausführungsbeispiel zwölf) Zähnen 70, die längs eines Kreises angeordnet sind und jeweils ein dreieckiges Zahnprofil haben.

An der Bodenplatte 60 des Körpers 52 ist im Bereich der ersten Anlagefläche 66 ein Gewindeschaft 72 ausgebildet, der mit einem Außengewinde 74 versehen ist. Der Gewindeschaft 72 ist von der kreisrunden ersten Zahnung 68 umgeben.

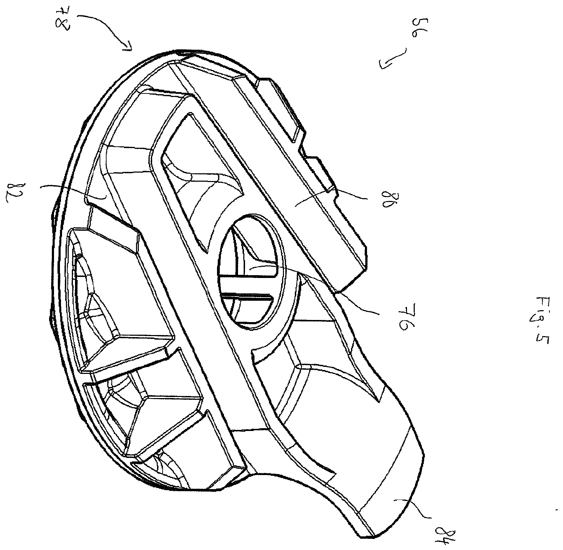

Die Aufnahmescheibe 56, die in Figur 5 nochmals in Alleinstellung dargestellt ist, hat ein mittiges, kreisrundes Durchgangsloch 76, über das die Aufnahmescheibe 56 auf den Gewindeschaft 72 steckbar ist. In dem Durchgangsloch 76 sind vier Raststifte 77 angeordnet, die jeweils an ihrem freien Ende eine radial nach innen gerichtete Rastnase 79 aufweisen (vgl. Figur 4). Wird die Aufnahmescheibe 56 auf den Gewindeschaft 72 gesteckt, so rasten die Rastnasen 79 in eine Rastrille 83 ein, die nahe der ersten Anlagefläche 66 ringförmig an dem Gewindeschaft 72 ausgebildet ist. Dadurch ist die Aufnahmescheibe 56 im aufgesteckten Zustand mit einer Kraft, die durch den elastischen Eingriff der Rastnasen 79 in der Rastrille 83 gegeben ist, nahe der Anlagefläche 66 gehalten und zugleich frei um den Gewindeschaft 72 drehbar. Die Raststifte 77 und die Rastrille 83 dienen so als Verliersicherung.

Wie am besten in Figur 5 zu sehen ist, hat die Aufnahmescheibe 56 eine Aufnahmenut 78, die aus zwei geraden Teilnuten 80 und 82 gebildet ist. Die Teilnuten 80 und 82 sind jeweils an ihren beiden Nutenden offen und so an der Aufnahmescheibe 56 ausgebildet, dass sich ihre Verlängerungen außerhalb der Aufnahmescheibe 56 in einem spitzen Winkel schneiden. Die Ausrichtung der beiden Teilnuten 80 und 82 entspricht der Ausrichtung der beiden Schenkel 110 und 112 des U-förmigen Abschnittes des Wundhakens 100 (vgl. Fig. 2). Die Teilnuten 80 und 82 sind ferner so angeordnet, dass sich das Durchgangsloch 76 zwischen den Teilnuten 80 und 82 befindet.

An der Aufnahmescheibe 56 ist ferner eine Nase 84 ausgebildet, die in radialer Richtung vom kreisrunden Rand der Aufnahmescheibe übersteht. Die Nase 84 schließt an die Teilnuten 80 und 82 an, und zwar an denjenigen Nutenenden, deren Verlängerungen auseinanderlaufen. Wie in Figur 2 gezeigt, dient die Nase 84 dazu, die Basis 108 des U-förmigen Abschnittes 102 des Wundhakens 100 zwischen sich und der Bodenplatte 60 des Körpers 52 aufzunehmen.

Wie in Figur 4 zu sehen, hat die Aufnahmescheibe 56 an ihrer Unterseite eine zweite Anlagefläche 86, die dazu bestimmt ist, mit der ersten Anlagefläche 66, die an der Oberseite der Bodenplatte 60 des Körpers 52 ausgebildet ist, in Presskontakt zu kommen. An der zweiten Anlagefläche 86 der Aufnahmescheibe 56 ist eine zweite umlaufende Zahnung 88 ausgebildet, die aus einer mehreren (im vorliegenden Ausführungsbeispiel zwölf) Zähnen 90 gebildet ist. Die Zähne 90 sind längs eines Kreises angeordnet, der das Durchgangsloch 86 umgibt. Wie die Zähne 70 der ersten Zahnung 68 haben auch die Zähne 90 der zweiten Zahnung 88 jeweils ein dreieckiges Zahnprofil. Die beiden Zahnungen 68 und 88 sind dazu bestimmt, ineinander zu greifen, wenn die Aufnahmescheibe 56 auf den Gewindeschaft 72 gesteckt ist und die beiden Anlageflächen 66 und 86 miteinander in Kontakt kommen. Liegt die Aufnahmescheibe 56 von dem Verriegelungselement 58 unbeaufschlagt auf der Bodenplatte 60 des Körpers 52 auf, so gibt es eine bestimmte Anzahl von stabilen Drehstellungen, in denen die beiden Zahnungen 68 und 88 ineinandergreifen. Diese Anzahl ist durch die Zahl von Zähnen 70 bzw. 90 der jeweiligen Zahnung 68 bzw. 88 bestimmt. Im vorliegenden Beispiel sind mithin zwölf stabile Drehstellungen vorgesehen. Der Winkel zwischen zwei benachbarten Drehstellungen beträgt dementsprechend 360°/12 gleich 30°. Selbstverständlich sind diese Angaben rein beispielhaft zu verstehen.

Das Verriegelungselement 58 weist ein Innengewinde 85 auf, das auf das Außengewinde 74 des Gewindeschafts 72 schraubbar ist. An dem Verriegelungselement 58 ein Sterngriff 87 ausgebildet, mit dem der Benutzer das Verriegelungselement 58 von Hand auf den Gewindeschaft 72 schrauben kann. Das Verriegelungselement 58 ist so ausgebildet, dass es die beiden Teilnuten 80 und 82 von oben bedeckt, wenn es auf den Gewindeschaft 72 geschraubt ist. Somit die beiden Schenkel 110 und 112 des Wundhakens 100, wenn sie in den Teilnuten 60 und 82 angeordnet sind, durch das Verriegelungselement 58 daran gehindert, sich aus den Teilnuten 80 und 82 zu lösen.

Um den Wundhaken 100 an dem Instrumentenhalter 50 zu befestigen, wird zunächst die Aufnahmescheibe 56 auf den Gewindeschaft 72 gesteckt. Anschließend wird der U-förmige Abschnitt 102 des Wundhaken 100 so an der Aufnahmescheibe 56 angebracht, dass die beiden Schenkel 110 und 112 des U-förmigen Abschnittes 102 des Wundhakens 100 in den beiden Teilnuten 80 und 82 aufgenommen werden und die Basis 108 des U-förmigen Abschnittes 102 unterhalb der Nase 84 und damit zwischen dieser und der Bodenplatte 60 des Körpers 52 angeordnet ist. Dann wird das Verriegelungselement 58 auf den Gewindeschaft 72 geschraubt. Hierzu greift der Benutzer den an dem Verriegelungselement 58 ausgebildeten Sterngriff 87 und zieht das Verriegelungselement 58, das sich mit seinem Innengewinde 85 im Gewindeeingriff mit dem Außengewinde 74 des Gewindeschafts 72 befindet, gegen die Aufnahmescheibe 56 fest. Ist das Verriegelungselement 58 festgezogen, so befinden sich die an der Bodenplatte 60 des Körpers 52 ausgebildete erste Anlagefläche 66 und die an der Aufnahmescheibe 56 ausgebildete zweite Anlagefläche 86 insbesondere im Bereich ihrer Zahnungen 68 und 88 im kraftschlüssigen Presskontakt, so dass die Aufnahmescheibe 56 und damit der in ihr gehaltene Wundhaken 100 in der gewünschten Stellung fixiert sind.

Soll die Schwenkstellung des Wundhakens 100 verändert werden, so löst der Benutzer die kraftschlüssige Verbindung zwischen den beiden Anlageflächen 66 und 86, indem er das Verriegelungselement 58 löst, ohne den Gewindeeingriff zwischen dem Verriegelungselement 58 und dem Gewindeschaft 72 vollständig aufzuheben. In diesem Zustand können die beiden Zahnungen 68 und 88 übereinander gleiten und in eine neue Stellung zueinander gebracht werden. Dementsprechend kann der Wundhaken 100 wie gewünscht verschwenkt werden.

In dem vorliegenden Ausführungsbeispiel ist das medizinische Instrument 100 ein Wundhaken. Es versteht sich von selbst, dass die Erfindung hierauf nicht beschränkt ist. So können auch Instrumente anderen Typs mit dem erfindungsgemäßen Instrumentenhalter 50 an dem Gelenkarm 10 gekoppelt werden.

### Bezugszeichenliste

- 10: Gelenkarm
- 12, 14, 16, 18, 20, 22: Halteglieder
- 24, 26, 28, 30, 32: Gelenke
- 36: Handgriff
- 38: Schnellkupplung
- 40: Entriegelungsknöpfe
- 50: Instrumentenhalter
- 52: Körper
- 54: Abdeckung
- 56: Aufnahmescheibe
- 58: Verriegelungselement
- 60: Bodenplatte
- 62: Rastplatte
- 64: Rastzapfen
- 66: erste Anlagefläche
- 68: Zahnung
- 70: Zähne
- 72: Gewindeschaft
- 74: Außengewinde
- 76: Durchgangsloch
- 77: Raststifte
- 78: Aufnahmenut
- 79: Rastnasen
- 80, 82: Teilnuten
- 83: Rastrille
- 84: Nase
- 85: Innengewinde
- 86: Durchgangsloch
- 87: Sterngriff
- 88: Zahnung
- 90: Zähne
- 100: Wundhaken
- 102: U-förmiger Abschnitt
- 104: Hakenabschnitt
- 106: Hakenblatt
- 108: Basis
- 110, 112: Schenkel

## Patentansprüche

1. Instrumentenhalter (50) zum Befestigen eines medizinischen Instrumentes (100) an einem Gelenkarm (10), mit:
einem Körper (52),
einer Halterung zum Halten des medizinischen Instrumentes (100) an dem Körper (52), und
einem Ankoppelelement (64) zum Anbringen des Körpers (52) an dem Gelenkarm (10), wobei die Halterung umfasst:
einen mit einem Außengewinde (74) versehenen Gewindeschaft (72), der von einer an dem Körper (52) ausgebildeten ersten Anlagefläche (66) absteht,
eine Aufnahmescheibe (56), die ein Durchgangsloch (76), über das die Aufnahmescheibe (56) auf den Gewindeschaft (72) steckbar und um den Gewindeschaft (72) drehbar ist, und eine zweiten Anlagefläche (86), die bei auf den Gewindeschaft (72) gesteckter Aufnahmescheibe (56) der ersten Anlagefläche (66) zugewandt ist, hat, **dadurch gekennzeichnet dass** die Aufnahmescheibe (56) eine Aufnahmenut (78) hat, die zur Aufnahme eines Teils (110, 112) des medizinischen Instrumentes (100) auf einer der zweiten Anlagefläche (86) abgewandten Seite der Aufnahmescheibe (56) ausgebildet ist, und
ein mit einem Innengewinde (85) versehenes Verriegelungselement (58), das im Gewindeeingriff seines Innengewindes (85) mit dem Außengewinde (74) des Gewindeschafts (72) die Aufnahmenut (78) zumindest teilweise bedeckt, wodurch der genannte Teil (110, 112) des medizinischen Instrumentes (100) in der Aufnahmenut (78) gehalten ist, und zur kraftschlüssigen Anlage der beiden Anlageflächen (66, 86) gegen die Aufnahmescheibe (56) festziehbar ist,
wobei an der Aufnahmescheibe (56) eine Nase (84) ausgebildet ist, die vom Rand der Aufnahmescheibe (56) seitlich übersteht und ausgebildet ist, einen U-förmigen Abschnitt (102) des medizinischen Instrumentes (100) zwischen sich und dem Körper (52) zu halten.

2. Instrumentenhalter (50) nach Anspruch 1, **dadurch gekennzeichnet, dass** die Aufnahmenut (78) aus zwei geraden, jeweils an beiden Nutenden offenen Teilnuten (80, 82) gebildet ist, wobei das Durchgangsloch (76) der Aufnahmescheibe (56) zwischen den beiden Teilnuten (80, 82) angeordnet ist.

3. Instrumentenhalter (50) nach Anspruch 2, **dadurch gekennzeichnet, dass** die beiden Teilnuten (80, 82) so angeordnet sind, dass sich ihre Verlängerungen außerhalb der Aufnahmescheibe (56) in einem spitzen Winkel schneiden.

4. Instrumentenhalter (50) nach einem der vorhergehenden Ansprüche, **dadurch gekennzeichnet, dass** das Verriegelungselement (58) einen Sterngriff (87) hat, mit dem das Verriegelungselement (58) von Hand auf den Gewindeschaft (72) schraubbar ist.

5. Instrumentenhalter (50) nach einem dem der vorhergehenden Ansprüche, **dadurch gekennzeichnet, dass** das Ankoppelelement eine an dem Körper (52) ausgebildete Rastnase (64) ist.

6. Instrumentenhalter (50) nach einem der vorhergehenden Ansprüche, **dadurch gekennzeichnet, dass** die erste Anlagefläche (66) eine erste Zahnung (68) und zweite Anlagefläche (86) eine zweite Zahnung (88) hat, die bei der Anlage der beiden Anlageflächen (66, 86) ineinandergreifen.

7. Instrumentenhalter (50) nach Anspruch 6, **dadurch gekennzeichnet, dass** die beiden Zahnungen (68, 88) jeweils mehrere längs eines Kreises angeordnete Zähne (70, 90) aufweisen, die jeweils ein dreieckiges Zahnprofil haben.

8. Instrumentenhalter (50) nach einem der vorhergehenden Ansprüche, **gekennzeichnet durch** mindestens ein in dem Durchgangsloch (76) der Aufnahmescheibe (56) angeordnetes erstes Rastelement (77, 79), das beim Aufstecken der Aufnahmescheibe (56) auf den Gewindeschaft (76) mit mindestens einem an dem Gewindeschaft (76) ausgebildeten zweiten Rastelement (83) in Eingriff kommt.

9. Medizinische Assistenzeinrichtung, umfassend einen Gelenkarm (10), mindestens ein medizinisches Instrument (100) und einen Instrumentenhalter (50) nach einem der vorhergehenden Ansprüche.

10. Medizinische Assistenzeinrichtung nach Anspruch 9, **dadurch gekennzeichnet, dass** das mindestens eine medizinische Instrument (100) eine Vielzahl von Instrumenten umfasst, die jeweils einen baugleichen, in der Aufnahmenut (78) des Instrumentenhalters (50) aufnehmbaren Teil (110, 112) aufweisen.

## Claims

1. An instrument holder (50) for fastening a medical instrument (100) to an articulated arm (10), with:
a body (52),
a holder for holding the medical instrument (100) on the body (52) and
a coupling element (64) for attaching the body (52) to the articulated arm (10),
wherein the holder comprises:
a threaded shaft (72) provided with an outer thread (74), which shaft projects from a first contact surface (66) formed on the body (52),
a receiving disk (56) which comprises a through hole (76) via which the receiving disk (56) can be inserted on the threaded shaft (72) and can rotate about the threaded shaft (72), and a second contact surface (86) which faces the first contact surface (66) when the receiving disk (56) is set on the threaded shaft (72), **characterized in that** the
receiving disk (56) comprises a receiving groove (78) which is designed to receive a part (110, 112) of the medical instrument (100) on a side of the receiving disk (56) facing away from the second contact surface (86), and
a locking element (58) provided with an inner thread (85) which element at least partially covers the receiving groove (78) in the threaded engagement of its inner thread (85) with the outer thread (74) of the threaded shaft (72)), as a result of which this part (100, 112) of the medical instrument (100) is held in the receiving groove (78) and can be tightened for a non-positive contacting of the two contact surfaces (66, 86) against the receiving disk (56),
wherein a nose (84) is formed on the receiving disk (56) and projects laterally from the edge of the receiving disk (56) and is designed to hold a U-shaped section (102) of the medical instrument (100) between itself and the body (52).

2. The instrument holder (50) according to Claim 1, **characterized in that** the receiving groove (78) is formed by two straight partial moves (80, 82) which are open at both groove ends,
wherein the through hole (76) of the receiving disk (56) is arranged between the two partial grooves (80, 82).

3. The instrument holder (50) according to Claim 2, **characterized in that** the two partial grooves (80, 82) are arranged in such a manner that their extensions intersect one another outside of the receiving disk (56) in an acute angle.

4. The instrument holder (50) according to one of the previous claims, **characterized in that** the locking element (58) has a star grip (87) with which the locking element (58) can be screwed manually onto the threaded shaft (72).

5. The instrument holder (50) according to one of the previous claims, **characterized in that** the coupling element is a catch nose (64) formed on the body (52).

6. The instrument holder (50) according to one of the previous claims, **characterized in that** the first contact surface (66) has a first toothing (68) and the second contact surface (86) has a second toothing (88) which engage in one another upon the contacting of the two contact surfaces (66, 86).

7. The instrument holder (50) according to Claim 6, **characterized in that** the two toothings (68, 88) comprise several teeth (70, 90) arranged along a circle which each have a triangular tooth profile.

8. The instrument holder (50) according to one of the previous claims, **characterized by** at least one first catch element (77, 79) arranged in the through hole (76) of the receiving disk (56) and which engages with at least one second catch element (83) formed on the threaded shaft (76) during the insertion of the receiving disk (56) on the threaded shaft (76).

9. A medical assistance device comprising an articulated arm (10), at least one medical instrument (100) and an instrument holder (50) according to one of the previous claims.

10. The medical assistance device according to Claim 9, **characterized in that** the at least one medical instrument (100) comprises a plurality of instruments which each comprise a structurally similar part (110, 112) which can be received in the receiving groove (78) of the instrument holder (50).

## Revendications

1. Porte-instrument (50) destiné à fixer un instrument médical (100) à un bras articulé (10), comprenant :
un corps (52),
un support pour tenir l'instrument médical (100) sur le corps (52), et un élément de couplage (64) destiné à attacher le corps (52) au bras articulé (10),
le support comprenant :
une tige filetée (72) dotée d'un filetage extérieur (74), qui fait saillie d'une première surface d'appui (66) formée sur le corps (52),
un disque de réception (56), qui a un trou de passage (76) par lequel le disque de réception (56) est enfichable sur la tige filetée (72) et pivotant autour de la tige filetée (72), et une deuxième surface d'appui (86), qui est tournée vers la première surface d'appui (66) lorsque le disque de réception (56) est étiré sur la tige filetée (72), **caractérisé en ce que** le disque de réception (56) a une rainure de réception (78), qui est formée sur un côté du disque de réception (56) opposé à la deuxième surface d'appui (86) en vue de réceptionner une partie (110, 112) de l'instrument médical (100), et un élément de verrouillage (58) doté d'un filetage intérieur (85), qui couvre au moins partiellement la rainure de réception (78) avec le filetage extérieur (74) de la tige filetée (72) dans la prise filetée de son filetage intérieur (85), ladite partie (110, 112) de l'instrument médical (100) étant maintenue dans la rainure de réception (78), et qui peut être serré contre le disque de réception (56) en vue d'une application de force des deux surfaces d'appui (66, 86),
un bec (84) étant formé sur le disque de réception (56), ledit bec faisant saillie latéralement du bord du disque de réception (56) et étant formé afin de maintenir une section en U (102) de l'instrument médical (100) entre lui et le corps (52).

2. Porte-instrument (50) selon la revendication 1, **caractérisé en ce que** la rainure de réception (78) est formée de deux rainures partielles (80, 82) droites ouvertes respectivement sur les deux extrémités de rainure, le trou de passage (76) du disque de réception (56) étant situé entre les deux rainures partielles (80, 82).

3. Porte-instrument (50) selon la revendication 2, **caractérisé en ce que** les deux rainures partielles (80, 82) sont disposées de telle manière que leurs prolongements se coupent suivant un angle aigu en dehors du disque de réception (56).

4. Porte-instrument (50) selon l'une quelconque des revendications précédentes, **caractérisé en ce que** l'élément de verrouillage (58) possède une poignée-étoile (87) avec laquelle l'élément de verrouillage (58) peut être vissé à la main sur la tige filetée (72).

5. Porte-instrument (50) selon l'une quelconque des revendications précédentes, **caractérisé en ce que** l'élément de couplage est un bec d'encliquetage (64) formé sur le corps (52).

6. Porte-instrument (50) selon l'une quelconque des revendications précédentes, **caractérisé en ce que** la première surface d'appui (66) possède une première denture (68) et la deuxième surface d'appui (86) une deuxième denture (88), qui s'imbriquent lors de la fixation des deux surfaces d'appui (66, 86).

7. Porte-instrument (50) selon la revendication 6, **caractérisé en ce que** les deux dentures (68, 88) présentent respectivement plusieurs dents (70, 90) disposées le long d'un cercle, lesdites dents ayant respectivement un profil de dent triangulaire.

8. Porte-instrument (50) selon l'une quelconque des revendications précédentes, caractérisé en au moins un premier élément d'encliquetage (77, 79) disposé dans le trou de passage (76) du disque de réception (56), ledit élément d'encliquetage vient en prise avec au moins un deuxième élément d'encliquetage (83) formé sur la tige filetée (76) pour insérer le disque de réception (56) sur la tige filetée (76).

9. Dispositif d'assistance médicale comprenant un bras articulé (10), au moins un instrument médical (100) et un porte-instrument (50) selon l'une quelconque des revendications précédentes.

10. Dispositif d'assistance médicale selon la revendication 9, **caractérisée en ce que** l'au moins un instrument médical (100) comprend une multitude d'instruments qui présentent respectivement une partie (110, 112) identique pouvant être réceptionnée dans la rainure de réception (78) du porte-instrument (50).
